(19) Europäisches Patentamt / European Patent Office / Office européen des brevets

(11) **EP 3 643 227 B1**

(12) **EUROPEAN PATENT SPECIFICATION**

(45) Date of publication and mention
of the grant of the patent:
**03.04.2024 Bulletin 2024/14**

(21) Application number: **19168944.7**

(22) Date of filing: **12.04.2019**

(51) International Patent Classification (IPC):
***A61B 5/024*** *(2006.01)*    ***A61B 5/11*** *(2006.01)*
***A61B 5/00*** *(2006.01)*

(52) Cooperative Patent Classification (CPC):
**A61B 5/024; A61B 5/1118; A61B 5/7221**

(54) **MINIMUM HEART RATE VALUE APPROXIMATION**

ANNÄHERUNG AN DEN MINIMALEN HERZFREQUENZWERT

APPROXIMATION D'UNE VALEUR DE RYTHME CARDIAQUE MINIMUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2018 US 201816171508**

(43) Date of publication of application:
**29.04.2020 Bulletin 2020/18**

(73) Proprietor: **Firstbeat Analytics Oy**
**40100 Jyväskylä (FI)**

(72) Inventors:
• **Korhonen, Joonas**
**40100 Jyväskylä (FI)**
• **Hämäläinen, Kaisa**
**40100 Jyväskylä (FI)**

(74) Representative: **Berggren Oy**
**P.O. Box 16**
**Eteläinen Rautatiekatu 10A**
**00101 Helsinki (FI)**

(56) References cited:
**WO-A1-2015/066430**    **WO-A1-2015/189304**
**WO-A1-2017/191085**

**Description**

Technical field

The application generally relates to heart-related measurements, and more particularly but not exclusively, the application relates to a minimum heart rate value approximation.

Background

Heart rate (HR) generally represents the number of contractions of the heart per minute (beats per minute or bpm). Heart rate may be monitored noninvasively by a wearable heart rate monitor. Heart rate variability (HRV) relates to the variation in the time intervals between individual heartbeats. The time between each successive heartbeat fluctuates depending on the situation. For example, the time interval between heartbeats increases during inspiration and decreases during expiration. The measured heart rate reactions and heart rate variability may be analysed in order to provide further information, for example on different bodily states of a user. Bodily states of a user may relate to stress, recovery and physical activity. Information on bodily states may be utilized widely to explore and improve well-being, health and performance.

WO 2015189304 discloses a heart rate monitoring system, which is able to determine a resting heart rate. Periods of inactivity of a user are also determined based on motion data.

Summary

It is an aim to provide accuracy to minimum heart rate value approximation based on collected heart rate data, without a need to wear a monitoring device continuously or during a sleep time of a user. For example, a user may wear the monitoring device during awake time only, and sleep without the wearable monitoring device. This enables implementing a user-friendly measurement, while providing improved accuracy to the analysis based on the collected heart rate data.

While continuous long term measurement may be advantageous for providing accurate results, battery operating time may pose limitations to continuously collecting data for a long term.

Aspects of the invention present a method, an apparatus, executable instructions and a computer program product for providing a minimum heart rate value approximation based on data collected during selected periods. The selected periods relate to inactivity periods of a user, while user is awake or not sleeping. The selected periods relate to immobile periods of a user, of a predetermined data quality, while a user is awake. The provided minimum heart rate value may be used for further analysis based on heart rate measurements.

Approximation of the minimum heart rate value, without requiring continuous 24-hours data collecting, provides user-friendliness, convenience and options for a user, for example relating to a time of wear and utilization of a monitor device.

A minimum heart rate is a physical characteristic of a person. The minimum heart rate represents the lowest heart rate a person can achieve, as opposite to maximal heart rate representing the highest heart rate a person can achieve. The minimum heart rate value approximation may enable for example accurate evaluation of normal physiological range of heart rate parameters for a given person, which may be utilized in various physiological analytics related to heart rate parameters as well as reflect person's health status and performance. A high minimum heart rate may reflect suboptimal health status of a person, such as elevated stress, blood pressure, or increased risk of cardiovascular diseases. A low minimum heart rate may be indicative to big size of heart, high stroke volume, and high parasympathetic modulation of the heart related to relaxed body state. Minimum heart rate value estimation may enable accurate evaluation of bodily states of a user and their intensity. A bodily state of recovery relates to low physical activity and for example low heart rate, while stress state relates to increased activation levels. Thus, the personal minimum heart rate may be used among other parameters to differentiate between stress and recovery (relaxation) states as it may be used to determine which measured heart rate level is in personal perspective low or high. Accordingly, accuracy of a minimum heart rate has an effect on accuracy on recognizing bodily states. The minimum heart rate value approximation may have effect on estimated intensity levels of a user, intensity of training, heart rate reserve and/or energy expenditure of activity.

A minimum heart rate, or minHR, may be approximated based on selected periods of collected heart rate data. Heart rate data may be detected over a predetermined or varying time intervals. If a detected data is qualified, the detected HR value and HR variability are recorded. The detected data qualifies, if user is awake, user is immobile and the data is of a predetermined quality. The predetermined data quality relates to quality of HR intervals and quality of HR levels, which may be free of artefacts and at a constant level. HR variability may correspond to a mean absolute difference/deviation (MAD). The values are added on corresponding previously detected values and the averages of them are calculated. An arithmetic, a weighted or other suitable average may be used. The minHR approximation may be implemented for on-line, i.e. real time measurement or data collecting, and/or existing data may be used off-line, i.e. after the data collection has ended.

An aspect of the invention comprises a method for providing an approximation of a minimum heart rate, (minHR) from collected heart rate data of a user. The method comprises calculating, from heartbeat signal collected from a user, a heart rate (HR) value and an artefact percentage for one or more time periods of the collected heartbeat signal; qualifying data of the time period(s) for which, the user is verified to be awake and immobile, and the artefact percentage is under a predetermined value, and disqualifying data of the other time periods; calculating heart rate parameters from the qualified data; and applying a function to the heart rate parameters in order to obtain the approximation of minHR.

Another aspect of the invention comprises an apparatus for providing an approximated minimum heart rate (minHR) from collected heart rate data of a user. The apparatus comprises an arrangement configured to calculate, from heartbeat signal collected from a user, a heart rate (HR) value and an artefact percentage for one or more time periods of the collected heartbeat signal; an arrangement configured to verify that the user is awake and immobile at the time period; an arrangement to calculate an artefact percentage for the time period; an arrangement configured to qualify data of the time period(s), if the user is verified to be awake and immobile, and if the artefact percentage is under a predetermined value; an arrangement configured to calculate heart rate parameters from the qualified data, and an arrangement for providing the approximation of minHR including applying a function to the heart rate parameters.

Still another aspect of the invention comprises an apparatus for providing a minimum heart rate value approximation, comprising executable instructions, which when executed by a processor, are arranged to implement: calculating, from heartbeat signal collected from a user, a heart rate (HR) value and an artefact percentage for one or more time periods of the collected heartbeat signal; qualifying data of the time periods for which, the user is verified to be awake and immobile, and the artefact percentage is under a predetermined value, and disqualifying data of the other time periods; calculating heart rate parameters from the qualified data, and applying a function to the heart rate parameters in order to obtain the approximation of minHR.

A yet another aspect of the invention comprises a computer program product for providing an approximated minimum heart rate, minHR, from collected heart rate data of a user. The computer program product comprising a processor, and a memory for storing program logic, wherein the program logic being executable by the processor. The program logic comprises: logic for calculating, from heartbeat signal collected from a user, a heart rate (HR) value and an artefact percentage for one or more time periods of the collected heartbeat signal; logic for qualifying the data of the time period(s), if the user is verified to be awake and immobile, and the artefact percentage is under a predetermined value, and disqualifying data of other time periods; logic for calculating heart rate parameters from the qualified data, and logic for applying a function to the heart rate parameters in order to obtain the approximation of minHR.

Description of the drawings

In the following embodiments are described in more detail with the accompanying drawings of which:

Figure 1      illustrates a method for providing information of user's bodily states based on collected heart rate data according to an embodiment.

Figure 2a      illustrates a method for approximating a minimum heart rate according to an embodiment.

Figure 2b      illustrates a method for an applied function according to an embodiment.

Figure 3      illustrates an apparatus for approximating a minimum heart rate according to an embodiment.

Figure 4      illustrates an apparatus for approximating a minimum heart rate according to an embodiment.

Detailed description

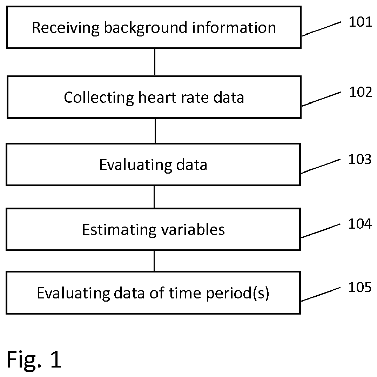

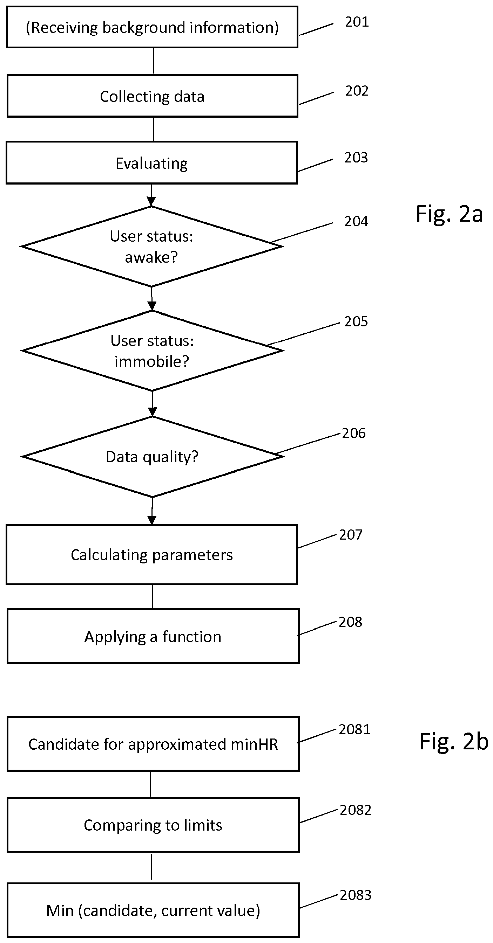

Figures 1 and 2 illustrate flowcharts for implementation of embodiments, which are disclosed herein. It is noted that the order of phases illustrated in Figures 1 or 2 is not required, and the various phases may be performed out of the illustrated order. In addition, certain phases may be skipped, different phases may be added or substituted, or selected phases or group of phases may be performed in a separate application, following the embodiments described herein.

Figure 1 illustrates a method for providing information of user's bodily states based on collected heart rate data according to an embodiment. A background information is provided 101. The background information may be inputted by a user or measured and/or inputted by an entity used for measuring and/or storing the background information, or fetched from such. The background information may include age, gender, height and weight of a user. Based on the background information estimates may be made. The estimates relate to user-specific values. For example, maximal heart rate ($HR_{max}$), maximal respiration rate and maximal oxygen consumption ($VO_{2max}$) may be estimated based on the background information. The maximal heart rate ($HR_{max}$) may be an age-based estimated maximal heart rate. The maximal oxygen consumption ($VO_{2max}$) may be estimated based on user information, like age, gender, height and weight. In addition, the maximal heart rate ($HR_{max}$) may have effect on maximal oxygen consumption ($VO_{2max}$). If values, like maximal heart rate, maximal oxygen consumption, activity class or other additional background information, are available, they may be inputted manually or automatically, as part of background information.

For the minimum HR approximation background information, as provided in phase 101 of Figure 1, is optional.

Heart rate data is collected 102. Beat-to-beat heart rate data may be collected in real-life settings over a desired period. The heart rate data may be collected with a heart rate monitor capable of measuring individual heartbeats. The collected beat-to-beat heart rate data may include time intervals between the heartbeats, being inter-beat intervals (IBI). Variation in the time interval between heartbeats is called heart rate variability (HRV). Individual range of physiological variables may be obtained from the heart rate variability (HRV) data. For example, maximal heart rate ($HR_{max}$) and resting heart rate ($HR_{rest}$) may be automatically updated in accordance to the collected data at phase 102. The maximal oxygen consumption ($VO_{2max}$) may be estimated based on collected heart rate and workload relationship from an exercise. If speed with altitude or power output is measured, for example via GPS, power meter or step rate sensor, maximal oxygen consumption ($VO_{2max}$) may be determined, for example as presented in US20140088444A1. The determined $VO_{2max}$ may be used to provide accuracy for minimum HR approximation.

The collected heart rate data is evaluated 103. Evaluation of data may comprise selecting, filtering and/or qualifying data for further use, or disqualifying data from the further analysis or use. The beat-to-beat heart rate data may be filtered in order to provide initial correction of artefact(s) comprising, falsely detected, missed and/or premature heartbeats. The collected heart rate data may be scanned through an artefact detection filter. If a difference between two consecutive heartbeats is over a predetermined limit, an error is detected. In order to avoid erroneous data to have effect on further analysis, the detected error or erroneous data may be removed or corrected. Error may be due to failure in measuring, malfunction of a measuring device, poor contact of the measuring equipment or irregular heartbeat of a user.

The consecutive filtered beat-to-beat heart rate intervals may be resampled. Resampling may be implemented by using linear interpolation, for example at a rate of 5 Hz. Resampling enables providing equidistantly sampled time series. After resampling low frequency trends and variances below and above a frequency band of interest may be removed from the resampled data. For example, a polynomial filter and/or a digital a finite impulse response (FIR) band-pass filter may be used.

Variables are estimated 104. Variables may be used for detecting physiological states. In addition to heart rate level, time domain and/or frequency domain of the heart rate variability may be provided as variables. The time domain of HR variability may comprise root mean square of successive heartbeat intervals. The frequency domain of HR variability may comprise high frequency power, low frequency power and amplitude of respiratory sinus arrhythmia. Values representing different physiological phenomena may be provided based on time or frequency domain variables. The physiological phenomena may be a respiration rate, oxygen consumption ($VO_2$) and/or an excess post-exercise oxygen consumption (EPOC). For example, the respiration rate may be derived from the collected heart rate data, for example as a beat-by-beat derived respiration rate. The HR and the respiration rate have a correlation with the oxygen consumption ($VO_2$). The oxygen consumption ($VO_2$) may be estimated based on collected heart rate data, the derived respiration rate and/or on/off response information derived from the collected heart rate data, such that an exercise intensity (%VO2max) may be calculated first, and the absolute oxygen consumption (ml/kg/min or ml/min) after that by multiplying intensity with person's $VO_{2max}$. Intensity may be calculated alternatively by dividing movement based $VO_2$ estimate by person's $VO_{2max}$. If the oxygen consumption ($VO_2$) is measured, respiration rate may not be needed at all, in which case, it may be that only the oxygen consumption ($VO_2$) and EPOC are used. Alternatively, all three may be utilized. The excess post-exercise oxygen consumption (EPOC) may be estimated based on the collected data of the heart rate measurement. The excess post-exercise oxygen consumption (EPOC) may be estimated based on intensity and duration of an exercise. The physiological phenomena may be used for detecting and recognizing different bodily states. Bodily state recognition may utilize given or pre-measured values of physiological phenomena.

Further analysis may be used to provide reliable results on bodily states. Segmentation may be utilized in off-line analysis. Segmentation may enable providing segments including physiologically coherent data. Segments may be categorized in order to provide reliable results on bodily states. The bodily states may relate to physical activity, to a recovery state, to a stress state and/or to any other bodily state. Some segments may remain unrecognized, without a detected bodily state. For on-line measurement, where the collected data is handled in real time, data points are handled instead of segments. A data point may represent a pre-determined or variable time period between successive data points. For example, a predetermined time period may be 5 seconds, or any predetermined time period between 3-60 seconds. The following applies to both off-line analysis, like for segmented data, and for on-line analysis, like for data points. Both, data points of online measurement and segments of off-line data relate to a certain time period.

Data of time period(s) may be evaluated 105 in order to identify different bodily states. Physical activity detection may be based on oxygen consumption ($VO_2$). For example, if oxygen consumption ($VO_2$) of data of a time period is more than a predetermined threshold (%) of the user's maximal oxygen consumption ($VO_{2max}$), data of the time period may be identified to represent physical activity. The predetermined threshold may relate to a metabolic value (MET) or to a percentage of $VO_{2max}$. For example, if oxygen consumption ($VO_2$) of data of a time period is over 7 ml/kg/min (2 METs), data of the time period may be identified to represent light-intensity exercise bout. On the other hand, exceeding 3MET and 40 % $VO_{2max}$ level may be regarded as a moderate intensity exercise bout and exceeding 60 % $VO_{2max}$ level may be regarded as a vigorous intensity exercise bout, for example. Physical activity detection may be based on data from a motion sensor. A motion sensor may be used as part of the heart rate monitor in order to provide information on movement. Motion sensor data may improve ability to recognize movements that are related to increased activation level of a user's body. This may replace or confirm physical activity detection based, at least partly, on oxygen consumption.

Physical activity detection may relate to an estimated post-exercise oxygen consumption (EPOC) value from a previously identified bout of exercise. If EPOC has reached a predetermined threshold value, which indicates an exercise session of a certain intensity of physical activity, data of subsequent time period(s) may be identified as representing a recovery period. This may be confirmed, if an excess post-exercise oxygen consumption (EPOC) is detected to decrease thereafter, at the following time period(s). Otherwise, if accumulation of EPOC still continues, exercise state may be determined to continue.

In addition to time period(s) relating to physical activity, time period(s) related to recovery state, stress state, an unrecognized state or any other state of a body may be detected. Recovery state detection may be based on a heart rate and a heart rate variability. If an individual heart rate is low and a heart rate variability is great, time period(s) may be identified as representing a recovery state. Minimum heart rates between individuals vary and a minimum heart rate is an individual, user and person dependent property. An individual minimum heart rate may be known, e.g. from previous measures during sleep time of a user, and/or it may be inputted as a background data. The collected heart rate(s) may be compared to the individual minimum heart rate, if the individual minimum heart rate is available. The individual minimum heart rate may have an effect on approximation of a minimum heart rate.

Stress state detection may be based on respiration rate, heart rate and/or heart rate variability variables, like high frequency power (HFP) and low frequency power (LFP). If an individual heart rate is elevated, a heart rate variability is decreased below individual basic resting level and/or a respiration rate is low compared to heart rate, time period (s) may be identified as representing a stress state. The detected heart rate(s) may be compared to an individual minimum heart rate in order to determine an individual elevated heart rate.

In addition to the ability to recognize bodily states, it is possible to recognize intensity of the states. Balance between stress and recovery states, moreover combined with intensity and duration of the states, provides information enabling exploring and improving well-being, health and performance. Accurate information is based on accurate measurements. However, continuous long-time measurement or data collecting is not always possible or desired. Measurement may be interrupted by a user or by a monitor device requiring service, for example recharging. For user convenience, collecting data during activity or awake time of a user may be preferred. In the following awake time of a user represents an identifiable state of a user, being separate from (opposite to) an identifiable time of sleep.

Data collecting only during awake time of a user may lead to inaccuracy of provided variables and/or lead to inaccuracy in identifying bodily states. For example, heart rate is lower during sleep time compared to awake time of a user. During sleep time external factors that may influence heart rate are reduced. Data collected only during awake or activity time of a user may lack accurate minimum heart rate value, which may be accurately measurable during sleep time of the user. Thus, data collected during sleep time may include a correct value of the minimum heart rate. Only few percent of data collected during awake time of a user may include minimum heart rate with an error margin of $\pm 1$ bpm of the correct minimum heart rate value. Thus, one aim is to provide accuracy to the minimum heart rate of a user without requiring sleep time measurements. Using an inaccurate value for minimum heart rate leads to inaccuracy for further analysis. For example, recognizing bodily states, intensity limits or intensity of training may be inaccurate due to use of an inaccurate minimum heart rate value. For example, the recognized amount of stress state may be underestimated, while the amount of recovery state may be overestimated. The under- and/or overestimated amounts of bodily states may differ 10-20% from the amounts identified using the correct minimum heart rate, collected during sleep time or rest time.

In order to provide accuracy to recognized bodily states based on data collected during awake/active time of a user, minimum heart rate may be approximated. The approximated minimum heart rate is based on heart rate data collected during awake and immobile time period(s) of a user. The time periods during which the user is awake and immobile may be verified. The verification of user's awakeness and immobility may be based on measured heart rate data, manually inputted data or the verification may be provided via other method(s) or means. The background information, like personal information of a user, may be optionally utilized. The minimum heart rate approximation may be based on data collected from a single day or from multiple days. At the minimum, one measurement (data point or segment) is required. However, the greater the amount of collected data, the greater the data set. The size of the data set may have an effect on reliability of the data. For online measurement time for collecting data may be, for example 2 hours or more. Shorter time, in order of minutes, may be sufficient for a control measurement, where the user is instructed to stay still during the control measurement. The control measurement may provide a reference estimation or a limit value for individual minimum HR.

Figure 2 illustrates a method for approximating a minimum heart rate from collected data. HR data is collected in order to provide approximation of the minHR. A HR value is calculated from the collected HR data. In addition, a HR variability may be calculated from the collected HR data.

Optionally, background information may be received 201. The background information may relate to a user-specific information, for example gender. If values, like maximal heart rate, maximal oxygen consumption, activity class or other additional background information, are available, they may be inputted manually or automatically, as part of background information. The background information may be inputted by a user or fetched from a storage medium.

Data is collected 202. If an existing data is used, data of a time period may be segmented. The segments may be handled and evaluated. If an on-line (real time) data collecting is established, data points representing a time period are detected. The time period may be a certain predetermined time period or variable time period. The time period may be from a few seconds to a minute or few minutes. For example, the time period may be 5 seconds, in which case data is collected once in every 5 seconds. A time period comprises heart rate data, from which at least a value for HR may be calculated. Optionally also HR variability may be calculated from the collected HR data.

The collected time period(s) are evaluated 203. The evaluation enables finding the selected periods that are qualified for further use or analysis. Data of the collected time period is qualified, if the user is awake 204, the user is immobile 205 and the data quality 206 is acceptable. The evaluation steps 204-206 may be implemented in any order or in parallel. If any one of the three is unacceptable or not true, the data of the disqualified time period is removed. Only data of the qualified time period(s) is saved. The saved data comprises HR value and an artefact percentage of the time period(s). The saved data may comprise HR variability of the time period(s).

Regarding data point evaluation, it is evaluated, if the data is collected during awake time of the user 204. It is not necessary for user to use a monitor/measurement device during sleep time. However, if an individual minimum heart rate during sleep time is recognized, it may be used for approximation as a limit value or candidate with data collected during awake time of the user. Awake time of a user may be identified from sleep time of the user. For example, motion sensor data, HR data and/or HRV data may be utilized. Motion sensor data represents amount of motion, and heart rate levels are decreased and heart rate variability is increased during sleep when compared to awake periods of a user. In addition or alternatively, awake time of a user may be based on other data, like manually inputted data or data provided by another method, or data fetched from another source.

A user may be verified to be awake based on at least one of: heart rate variability levels, deviation of heart rate variability, heart rate level, variability of heart rate level, the current minimum heart rate value, motion data, user input and data provided by other sources.

HR value and an artefact percentage, as well as HR variation, which is calculated from data collected during awake time of the user, may be saved for further use, if it qualifies the other evaluation criteria (204-206). If the data is collected during non-awake or sleep time of a user, it is not saved.

It is evaluated, if the data collecting is implemented during immobility of the user 205. Data collected during an awake time of a user comprises mobile and immobile periods. The collected data of a time period is evaluated in order to find collected data during an immobile period. Immobile period(s) may be detected from lack of movement, low oxygen consumption ($VO_2$) levels and low post-exercise oxygen consumption (EPOC). Immobile periods may be recognized from heart rate quality and motion data. Lack of movement may be detected via movement sensors including one or more of accelerometer, 3-axis accelerometer, gyroscope, GPS. Recognizing immobility or lack of movement may comprise detected movement below a predetermined threshold value, for example of less than 2 km/h. A state detection algorithm may be used for identifying immobile periods. For example, if the user is found to exercise or recover from an exercise, the data is not saved. HR value and an artefact percentage, as well as HR variation, which is calculated from data collected during an immobile period of the user may be saved for further use, if it qualifies the other evaluation criteria (204-206). If the data is collected during a mobile period of a user, it is not saved.

A user may be verified to be immobile based on at least one of: motion data, oxygen consumption ($VO_2$) levels, post-exercise oxygen consumption (EPOC), percentage of maximal oxygen consumption ($\%VO_{2max}$), and physical activity state.

Those may comprise heart rate based estimate on percentage of maximal oxygen consumption ($\%VO_{2max}$), and heart rate based physical activity state may be estimated based on heart rate.

Oxygen consumption ($VO_2$) levels, post-exercise oxygen consumption (EPOC), percentage of maximal oxygen consumption ($\%VO_{2max}$), and physical activity state may be estimated based on heart rate.

The oxygen consumption ($VO_2$) levels may be estimated using heart rate and motion data. The percentage of maximal oxygen consumption ($\%VO_{2max}$) may be estimated using heart rate and motion data.

A user may be distinguished as being awake and mobile using threshold values based on heart rate and heart rate variability data, and heart rate difference from its minimum. The threshold values for both movement and heart rate are based on the currently estimated minimum heart rate data and corresponding movement threshold values that express whether there is significant movement or not. Alternatively, a neural network may be utilized to distinguish awake periods where movement activity, heart rate and/or heart rate variable inputs may be used. The neural network may be trained to distinguish when a person is sleeping and when they are awake. The training may be implemented with classified HRV (R-R intervals) and accelerometer data.

It is evaluated, if the collected data is of a good quality 206. In order to qualify data of a time period being of good quality, short term and long term artefact percentages of the collected data values, like HR, shall be low enough. Threshold values may be predetermined. For example, values differing too much from previously saved values may be disqualified. In addition, HR level may be estimated and required to have certain stability. For example deviation of more than 10 bpm between successive segments/data points may not be allowed. Possible artefacts and/or deviations may be identified from short time periods, e.g. successive time periods, as well as from longer time collected averaged values. Artefact percentage may not exceed a predetermined threshold value, for example 30%. HR value and an artefact percentage, as well as HR variation, which is calculated from collected data of good quality may be saved for further use, if it qualifies the other evaluation criteria (204-206). If the data is not of good quality, for example comprises artefacts or inconstant HR levels, it is not recorded.

Values calculated from data of the time period(s), which is qualified based on evaluation, may be saved or recorded to a database. Single values and average of all the qualified values may be saved. The values may be HR, artefact percentage and HR variability.

Heart rate parameters are calculated 207 from the qualified data. Heart rate parameters may include an average, standard deviation, minimum and maximum of heart rate (HR) and/or HR variability (HRV).

A function is applied 208 to the heart rate parameters. The function is applied in order to obtain a minHR approximation. As a result of applying a function to the HR parameters, a minHR approximation is provided.

The function may include a mathematical function or a model, a multivariable model, a regression model, a linear model or a multivariable regression model, for example. The function may be applied to at least one or more of the following HR parameters collected during immobile periods: an average heart rate level, standard deviation of heart rate, an average level of HR variability and a minimum heart rate. Used function may be adapted by lifestyle assessment data. In addition to the HR parameters, user inputted behaviour and perceptions may be taken into account in the function. User input may comprise user perceived sleep quality, user perceived stress level, user perceived well-being, or user reported alcohol use, for example.

The approximation of Figure 2a may continue to evaluate 203 data of the next data point or segment. The evaluation 203 including qualifying or disqualifying the data point/segment is based on whether user has been awake 204 and immobile 205 and whether the data quality 206 is acceptable. HR parameters are calculated form the qualified data 207. A function is applied to the parameters 208. The function may comprise adding a constant to the data, comparing with upper and lower limit values, and/or selecting minimum of: the evaluated value for minHR and the current candidate for the approximated minHR value in order to find the minimum as the approximated minHR. In a segmented off-line solution, the approximated minHR may be established after all segments of a completed measurement of a user have been processed or after certain number of segments have been processed. For online application, a certain number of data points may be processed, or processed data points may be collected over a certain or non-predetermined measurement time.

An approximation of minimum heart rate value provides accuracy to the provided minHR value. It is possible to remove for example a percentile of collected values in order to remove the ones differing the most from an average. Approximation may include upper and lower limit values for the approximated minimum heart rate. It is possible to use the smallest collected value, in case the collected value is smaller than the candidate for the approximated value. The comparisons and selections may provide further accuracy to the approximations. Over- and underestimations of the approximated minimum heart rate may be decreased or minimized.

Figure 2b illustrates an applied function according to an embodiment. The function applied to the HR parameters may comprise adding a constant may to the resulting value(s) of the function. The constant may be added in order to avoid underestimation. Some rules and/or threshold may be predetermined for adding a constant. For example, it may be determined that in database level only 5 % of saved data is underestimated more than 1 bpm.

A function may comprise obtaining a candidate for approximated minHR 2081. An average of the saved, qualified minimum heart rate values may be calculated. Values of good quality from multiple successive time periods are used for calculating the average. The calculated average value may be selected as a candidate for an approximated minimum heart rate.

A function for calculating a candidate for approximated minHR (E) may be

$$E = w_{HR_{avg}} \cdot HR_{avg} + w_{HR_{std}} \cdot HR_{std} + w_{HR_{min}} \cdot HR_{min} + w_g \cdot g + C,$$

where $HR_{avg}$ is the average heart rate, $HR_{std}$ is the standard deviation of heart rate, and $HR_{min}$ is the minimum heart rate, each calculated from the accepted data points. $g$ is the gender, which may be a selected integer, for example 1 for women and 2 for men. $w_x$ is the coefficient of the corresponding variable x and C is a constant.

The candidate value for an approximated minHR may be limited 2082 between selected upper and lower limits. The candidate for an approximated minimum heart rate value may be compared with a predetermined upper limit value for a minimum heart rate value. The predetermined upper limit value for a minimum heart rate may be from 60 to 90 bpm. The smaller one of the compared values is selected to be used as a candidate for an approximated minimum heart rate value. This may have the effect of avoiding overestimation of the approximated minimum heart rate value. Similarly, a lower limit may be formed by detected minimum heart rate value or it may be a predetermined value, for example 35-40 bpm. The candidate value for an approximated minHR is compared with the lower limit, and the larger of them is selected as a candidate for an approximated minimum heart rate value. This may have the effect of providing certain predetermined minimum value for a candidate for an approximated minHR. This may avoid underestimations of the approximated minimum HR value.

The approximated minHR value is obtained 2083. In the function of Figure 2b, the minimum of the detected minHR and the current candidate for the approximated minHR value is selected as the approximated minHR. The detected value for minHR is based on values of good quality data from multiple successive time periods. In case, from daytime measurements, a detected minHR is lower than the approximated minHR, the detected minHR is selected.

Accuracy of the approximated minHR may be measured with mean absolute error (MAE), which may be defined as follows:

$$MAE = \frac{1}{N} \sum_{j=1}^{N} |x_j - y_j|,$$

where N is the number of values, $x_j$ is the jth estimated value, and $y_j$ is the jth correct value. In this case the correct values are minHR from sleep data. The approximated minHR may have MAE of below 5.0 bpm, for example 4.4 bpm; whereas the detected daytime minHR may have MAE of over 6.0 bpm.

Provided better accuracy in approximated minHR may have significant effect to accuracy of recognizing bodily states or estimating intensity of physical activity. For example, bodily states of stress and recovery may be recognized more accurately. Approximated minHR may provide more accuracy to estimations of intensity limits of a user, intensity of training, current intensity, what a user is doing at a given moment, and/or active moments of a user. Approximated minHR may provide accuracy to calculation of metabolic value, MET and/or heart rate reserve, HRR, and/or maximum oxygen consumption, VO2max. Approximated minHR may provide accuracy to any results and estimations using minHR as a parameter or variable. For example, if minimum heart rate is based on awake data, i.e. not using either the true minHR value, nor approximation, utilization of the minHR may cause error in stress and relaxation percentages for any day when compared to results obtained using true minHR. This error may be significantly reduced when utilizing the approximation as disclosed in this application.

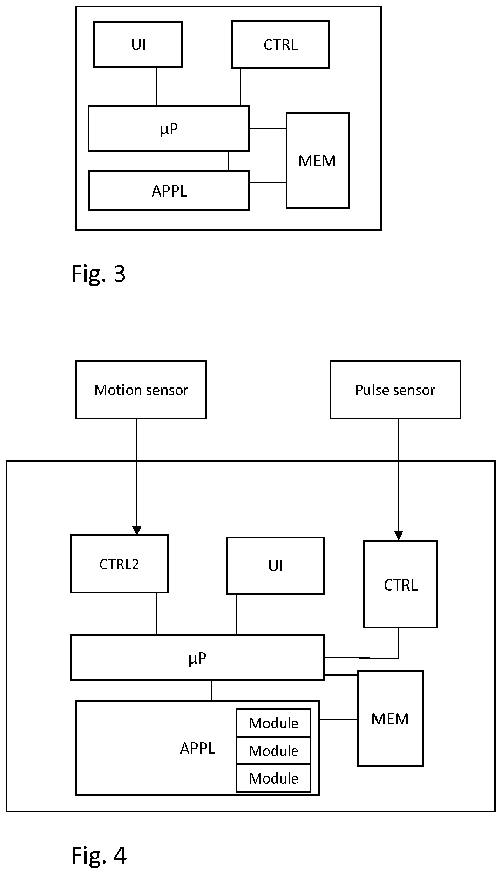

Figure 3 illustrates an apparatus for providing a minimum heart rate approximation. Figure 3 shows a simplified block diagram of the apparatus. The apparatus comprises a minimum heart rate approximation application module APPL, at least one processor µP, at least one memory MEM and a user interface module UI. The at least one memory MEM is configured to store or record information and executable instructions. These include, for example, user background information, executable instructions configured to provide a minimum heart rate approximation, executable instructions configured to calculate/compare variables, executable instructions configured to detect/select immobile periods and variables, executable instructions configured to analyse measured HR data, e.g. HR and HR variability. The executable instructions may comprise executable instructions configured to determine intensity levels or limits and/or executable instructions configured to recognize bodily states, for example as illustrated with methods of Figures 1 or 2. A user interface UI is configured to receive information inputted by a user and to present information. The user interface UI may be used to receive inputted information, like user background information and/or to present information, like presenting information to a user.

Various embodiments of at least one memory MEM may include any suitable data storage technology type, including but not limited to semiconductor based memory devices, magnetic memory devices and systems, optical memory devices and systems, fixed memory, removable memory, disc memory, flash memory, non-transitory computer readable memory, dynamic random access memory (DRAM), static random access memory (SRAM), electrically erasable programmable read-only memory (EEPROM) and alike.

Various embodiments of the processor μP include, but are not limited to, general purpose computers, special purpose computers, microprocessors, digital signal processors (DSPs) and multi-core processors.

The apparatus of figure 3 may comprise a processor μP and computer executable instructions stored in a memory MEM, which are arranged to implement the approximation of minimum heart rate from collected data. The apparatus of figure 3 may comprise a hardware, like an electric circuit, an application specific integrated circuit (ASIC), a field-programmable gate arrays (FPGA), a microprocessor coupled with memory that stores instructions executable by the microprocessor.

The apparatus of figure 3 may comprise a controller CTRL, or a heart rate module, configured to receive a signal from a pulse sensor or a heart rate sensor. The minimum heart rate approximation module APPL may be configured to process the received signal, with aid of the microprocessor μP and data stored/recorded to the memory MEM. The minimum heart rate approximation module APPL may comprise at least an approximated minimum heart rate calculation module.

The minimum heart rate approximation module APPL of figure 3 is configured to process collected data, for example to qualify or disqualify data, to record qualified data, to model data with a linear model, to compare data, and to provide approximation of minHR. The minHR approximation module APPL may be implemented as an application computer program stored in a memory, for example to the at least one memory MEM.

In addition to modules presented in figure 3, figure 4 illustrates a controller CTRL2, a pulse sensor and a motion sensor configured to provide signals to corresponding controllers CTRL, CTRL2, and modules of the minimum heart rate approximation module APPL.

The apparatus of figure 4 may comprise a controller CTRL, or a heart rate module, configured to receive a signal from a pulse sensor or a heart rate sensor. The heart rate sensor may be attached to a user. An (external) apparatus configured to monitor heart rate may comprise a monitor device, a heart rate monitor, a pulse rate monitor, a biometric device, a personal monitor, a portable monitor or a wearable monitor. The apparatus may comprise a physiological sensor, like an optical reflectometer. The apparatus, like a heart rate monitor, enables measuring and/or monitoring heart rate of a user and providing the measured signal(s) to a controller CTRL or a heart rate module.

The apparatus of figure 4 may comprise another controller CTRL2, or a motion sensor module, configured to receive a signal from a motion sensor. An (external) apparatus configured to monitor motion may comprise a motion sensor, a motion detector, an accelerometer, an inertial sensor, a gyroscopic sensor, or other sensor arranged to detect movement. A motion sensor may be an integral part of a heart rate monitor. The apparatus, like a portable monitor device, is arranged to collect data from embedded sensors, for example a heart rate sensor and a motion sensor. The portable monitor apparatus may communicate with an external device or a server. The portable monitor apparatus may communicate or relay the collect data to other entity. The entity receiving data from the portable monitor apparatus may comprise modules of apparatus of figure 4. Apparatus of figure 4 and figure 3 may comprise a web service, a computer, a mobile phone, a server for storing and processing the data. The communication may be realized via wired or wireless communications.

The minimum heart rate approximation module APPL of figure 4 comprises dedicated modules configured to process collected data, for example to qualify or disqualify data, to record qualified data, to model data with a linear model, to compare data, and/or to provide approximation of minHR. The minHR approximation module APPL may be implemented as an application computer program stored in a memory, for example to the at least one memory MEM. The minimum heart rate approximation module APPL, or dedicated modules of it, may be implemented at least partly as a software, a firmware and/or a hardware module or a combination thereof. In the case of the software or firmware, an embodiment may be implemented using a software related product such as a computer readable memory, for example a non-transitory computer readable memory, computer readable medium or a computer readable storage structure comprising computer readable instructions, for example program instructions, using a computer program code, like a software or a firmware, thereon to be executed by a computer processor.

The apparatus of figure 4, a motion sensor module CTRL2, a heart rate module CTRL, and/or an approximated minimum heart rate calculation module APPL may be implemented as a separate block/module, or may be combined with any other block/module of the apparatus of figure 4, or may be distributed into several blocks/modules according to their functionality. Moreover, it is noted that all or selected modules of the apparatus figures 3 and 4 may be implemented using an integral circuit, for example an application specific integrated circuit (ASIC).

An apparatus according to aspects of the invention may comprise an arrangement configured to obtain a candidate for a minHR and a lowest calculated HR value from the qualified time periods of more than two successive time periods, and an arrangement configured to select the minimum of: the candidate for a minHR and the lowest calculated HR value, as the approximation of minHR.

An apparatus according to aspects of the invention may comprise the heart rate data including inter-beat interval data and an arrangement configured to calculate heart rate variation (HRV) based on the inter-beat interval data, and the HR parameters comprising at least one of: an average of HRV, a standard deviation of HRV, a minimum value of HRV and a maximum value of HRV.

An apparatus according to aspects of the invention may comprise an arrangement configured to verify that the user is awake based on at least one of: heart rate variability levels, deviation of HR variability, user input, data provided by other source.

An apparatus according to aspects of the invention may comprise an arrangement configured to verify that the user is immobile based on at least one of: motion data, oxygen consumption ($VO_2$) levels, post-exercise oxygen consumption (EPOC), percentage of maximal oxygen consumption ($\%VO_{2max}$) and physical activity state. The motion data, oxygen consumption ($VO_2$) levels, post-exercise oxygen consumption (EPOC), percentage of maximal oxygen consumption ($\%VO_{2max}$) and/or physical activity state heart rate may be estimated based on heart rate data.

An apparatus according to aspects of the invention may comprise an arrangement configured to calculate an artefact percentage from at least one of: previous time period and averaged values of the sequential time periods for the time period.

An apparatus according to aspects of the invention may comprise an arrangement configured to calculate an artefact percentage and to compare HR values, wherein optionally a threshold value is predetermined, or based on difference compared to a previous value(s), or based on deviation of data between successive time periods.

An apparatus according to aspects of the invention may comprise at least one of: an arrangement configured to receive heart rate data of a user from sequential time periods online; and an arrangement configured to receive previously collected heart rate data of a user.

An apparatus according to aspects of the invention may comprise a processor, and a memory for storing the arrangements comprising a program logic, wherein the program logic being executable by the processor.

Unless otherwise defined, technical and scientific terms used herein have the same meaning as is commonly understood by one having ordinary skill in the art to which this disclosure belongs. The terms "first", "second", and the like, as used herein do not denote any order, quantity, or importance, but rather are employed to distinguish one element from another. Also, the terms "a" and "an" do not denote a limitation of quantity, but rather denote the presence of at least one of the referenced items. The use of "including", "comprising" or "having" and variations thereof herein are meant to encompass the items listed thereafter and equivalents thereof, as well as additional items. The terms "including", "comprising" or "having" and variations thereof inherently consist of the items listed thereafter and equivalents thereof. The terms "connected" and "coupled" are not restricted to physical or mechanical connections or couplings, and may can include electrical or optical connections or couplings, whether direct or indirect.

Furthermore, the skilled artisan will recognize the interchangeability of various features or parts from different embodiments. The various features or parts described, as well as other known equivalents for each feature, can be mixed and matched by one of ordinary skill in this art, to construct additional systems and techniques in accordance with principles of this disclosure.

It is to be understood that the foregoing description is intended to illustrate and not to limit the scope of the invention, which is defined by the scope of the appended claims. Other embodiments are within the scope of the following claims. The foregoing description should therefore be considered as merely illustrative of the principles, teachings and embodiments of this invention, and not in limitation thereof.

**Claims**

1. A computer-implemented method for providing an approximation of a minimum heart rate (minHR) from collected heart rate data of a user, the method comprising

   a) calculating, from heartbeat signal collected from a user, a heart rate (HR) value and an artefact percentage for one or more time periods of the collected heartbeat signal,
   b) qualifying data of the time period(s) for which,

   o the user is verified to be awake and immobile, wherein the user is verified to be awake based on at least one of: heart rate variability levels, deviation of HR variability, heart rate level, variability of heart rate level, the current minimum heart rate value, motion data, user input and data provided by other source, and wherein the user is verified to be immobile based on at least one of motion data, oxygen consumption (VO2) levels, post-exercise oxygen consumption (EPOC), percentage of maximal oxygen consumption (%VO2max) and physical activity state, and
   o the artefact percentage is under a predetermined value, wherein the artefact percentage comprises comparison of inter-beat interval values, wherein optionally a threshold value is predetermined, or based on difference compared to a previous value(s), or based on deviation of data between successive time periods,

and disqualifying data of the other time periods,

c) calculating heart rate parameters from the qualified data,

d) applying a function to the heart rate parameters in order to obtain the approximation of minHR, wherein the function comprises obtaining a candidate for a minHR and a lowest calculated HR value from the qualified time periods of more than two successive time periods, and selecting the minimum of: the candidate for a minHR and the lowest calculated HR value as the approximation of minHR.

2. A method according to any of the preceding claims, wherein HR parameters comprise at least one of average, standard deviation, minimum value and maximum value of HR.

3. A method according to any of the preceding claims, wherein heart rate data comprises inter-beat interval data and the method comprises calculating heart rate variation (HRV) based on the inter-beat interval data, and the HR parameters comprise at least one of: an average of HRV, a standard deviation of HRV, a minimum value of HRV and a maximum value of HRV.

4. A method according to any of preceding claims, wherein the function comprises predetermined parameters for upper and lower limits for the HR value and HR variability; and optionally limiting the minHR candidate between the predetermined upper and lower limits.

5. A method according to any of preceding claims, comprising receiving background information of a user, wherein background information comprises user specific information, optionally age and gender.

6. A method according to any of preceding claims, wherein artefact percentage is determined from previous time period and/or from averaged values of the sequential time periods.

7. A method according to any of preceding claims, wherein the heart rate data from a user comprises collecting data from sequential time periods points via online measurement.

8. A method according to any of preceding claims, wherein the heart rate data from a user comprises saved, previously collected data, optionally segmented data.

9. An apparatus for providing an approximated minimum heart rate (minHR) from collected heart rate data of a user, the apparatus comprising

- an arrangement configured to calculate, from heartbeat signal collected from a user, a heart rate (HR) value and an artefact percentage for one or more time periods of the collected heartbeat signal,

- an arrangement configured to verify that the user is awake and immobile at the time period, wherein the user is verified to be awake based on at least one of: heart rate variability levels, deviation of HR variability, heart rate level, variability of heart rate level, the current minimum heart rate value, motion data, user input and data provided by other source, and wherein the user is verified to be immobile based on at least one of motion data, oxygen consumption (VO2) levels, post-exercise oxygen consumption (EPOC), percentage of maximal oxygen consumption (%VO2max) and physical activity state,

- an arrangement configured to calculate an artefact percentage for the time period, wherein the artefact percentage comprises comparison of inter-beat interval values, wherein optionally a threshold value is predetermined, or based on difference compared to a previous value(s), or based on deviation of data between successive time periods,

- an arrangement configured to qualify data of the time period(s), if the user is verified to be awake and immobile; and if the artefact percentage is under a predetermined value,

- an arrangement configured to calculate heart rate parameters from the qualified data, and

- an arrangement configured to apply a function to the heart rate parameters, in order to obtain the approximation of minHR, wherein the function comprises obtaining a candidate for a minHR and a lowest calculated HR value from the qualified time periods of more than two successive time periods, and selecting the minimum of: the candidate for a minHR and the lowest calculated HR value as the approximation of minHR.

10. An apparatus according to claim 9, comprising one or more arrangement(s) configured to implement the method according to any of claims 2-8.

11. A computer program product for providing a minimum heart rate value approximation, comprising executable instructions, which when executed by a processor, are arranged to implement the method according to any of claims 1-8.

**Patentansprüche**

1. Computer-implementiertes

   Verfahren zur Bereitstellung einer Annäherung an eine minimale Herzfrequenz (minHF) aus gesammelten Herzfrequenzdaten eines Benutzers, wobei das Verfahren Folgendes umfasst
   a) Berechnen eines Herzfrequenzwertes (HF) und eines Artefakt-Prozentsatzes für eine oder mehrere Zeitperioden des gesammelten Herzfrequenzsignals aus dem Herzschlagsignal eines Benutzers,
   b) Qualifizierung der Daten der Zeitperiode(n), für die,

   o der Benutzer als wach und unbeweglich verifiziert wird, wobei der Benutzer auf der Grundlage von mindestens einem der folgenden Elemente als wach verifiziert wird: Herzfrequenzvariabilitätsniveaus, Abweichung der Herzfrequenzvariabilität, Herzfrequenzniveau, Variabilität des Herzfrequenzniveaus, aktueller minimaler Herzfrequenzwert, Bewegungsdaten, Benutzereingaben und Daten, die von einer anderen Quelle bereitgestellt werden, und wobei der Benutzer auf der Grundlage von mindestens einem der folgenden Elemente als unbeweglich verifiziert wird: Bewegungsdaten, Sauerstoffverbrauchsniveau (VO2), Sauerstoffverbrauch nach einem Training (EPOC), Prozentsatz des maximalen Sauerstoffverbrauchs (%VO2max) und körperlicher Aktivitätszustand, und
   o der Artefakt-Prozentsatz unter einem vorbestimmten Wert liegt, wobei der Artefakt-Prozentsatz den Vergleich von Interbeat-Intervallwerten umfasst, wobei optional ein Schwellenwert vorbestimmt ist, oder auf der Differenz im Vergleich zu einem oder mehreren vorherigen Werten basiert, oder auf der Abweichung von Daten zwischen aufeinanderfolgenden Zeitperioden basiert,

   und Ausschluss von Daten aus den anderen Zeitperioden,
   c) Berechnen von Herzfrequenzparametern aus den qualifizierten Daten,
   d) Anwenden einer Funktion auf die Herzfrequenzparameter, um die Annäherung der minHF zu erhalten, wobei die Funktion das Erhalten eines Kandidaten für eine minHF und eines niedrigsten berechneten HR-Wertes aus den qualifizierten Zeitperioden von mehr als zwei aufeinanderfolgenden Zeitperioden und das Auswählen des Minimums von: dem Kandidaten für eine minHF und dem niedrigsten berechneten HF-Wert als die Annäherung der minHF umfasst.

2. Ein Verfahren nach einem der vorhergehenden Ansprüche, wobei die HR-Parameter mindestens einen der folgenden Werte umfassen: Durchschnitt, Standardabweichung, Minimalwert und Maximalwert der HF.

3. Ein Verfahren nach einem der vorhergehenden Ansprüche, wobei die Herzfrequenzdaten Interbeat-Intervalldaten umfassen und das Verfahren die Berechnung der Herzfrequenzvariation (HFV) auf der Grundlage der Interbeat-Intervalldaten umfasst, und die HF-Parameter mindestens eines der folgenden Merkmale umfassen: einen Durchschnitt der HFV, eine Standardabweichung der HFV, einen Minimalwert der HFV und einen Maximalwert der HFV.

4. Ein Verfahren nach einem der vorhergehenden Ansprüche, wobei die Funktion vorbestimmte Parameter für obere und untere Grenzen für den HF-Wert und die HF-Variabilität umfasst; und optional die Begrenzung des minHF-Kandidaten zwischen den vorbestimmten oberen und unteren Grenzen.

5. Ein Verfahren nach einem der vorhergehenden Ansprüche, umfassend das Empfangen von Hintergrundinformationen eines Benutzers, wobei die Hintergrundinformationen benutzerspezifische Informationen, optional Alter und Geschlecht, umfassen.

6. Ein Verfahren nach einem der vorhergehenden Ansprüche, wobei der Artefakt-Prozentsatz aus der vorhergehenden Zeitperiode und/oder aus den gemittelten Werten der aufeinanderfolgenden Zeitperioden bestimmt wird.

7. Ein Verfahren nach einem der vorhergehenden Ansprüche, wobei die Herzfrequenzdaten eines Benutzers das Sammeln von Daten aus aufeinanderfolgenden Zeitperioden über eine Online-Messung umfassen.

8. Ein Verfahren nach einem der vorhergehenden Ansprüche, wobei die Herzfrequenzdaten eines Benutzers gespeicherte, zuvor gesammelte, optional segmentierte Daten, umfassen.

9. Eine Vorrichtung zum Bereitstellen einer angenäherten minimalen Herzfrequenz (minHF) aus gesammelten Herzfrequenzdaten eines Benutzers, wobei die Vorrichtung Folgendes umfasst

- eine Anordnung, die so konfiguriert ist, dass sie ein Herzfrequenzwert (HF) und ein Artefakt-Prozentsatz für eine oder mehrere Zeitperioden des gesammelten Herzfrequenzsignals aus dem Herzschlagsignal eines Benutzers berechnet,
- eine Anordnung, die so konfiguriert ist, dass sie verifiziert, dass der Benutzer zu der Zeitperiode wach und unbeweglich ist, wobei der Benutzer auf der Grundlage von mindestens einem der folgenden Elemente als wach verifiziert wird: Herzfrequenzvariabilitätsniveaus, Abweichung der Herzfrequenzvariabilität, Herzfrequenzniveau, Variabilität des Herzfrequenzniveaus, aktueller minimaler Herzfrequenzwert, Bewegungsdaten, Benutzereingaben und Daten, die von einer anderen Quelle bereitgestellt werden, und wobei der Benutzer auf der Grundlage von mindestens einem der folgenden Elemente als unbeweglich verifiziert wird: Bewegungsdaten, Sauerstoffverbrauchsniveau (VO2), Sauerstoffverbrauch nach einem Training (EPOC), Prozentsatz des maximalen Sauerstoffverbrauchs (%VO2max) und körperlicher Aktivitätszustand,
- eine Anordnung, die so konfiguriert ist, dass sie einen Artefakt-Prozentsatz für die Zeitperiode berechnet, wobei der Artefakt-Prozentsatz den Vergleich von Interbeat-Intervallwerten umfasst, wobei optional ein Schwellenwert vorbestimmt ist, oder auf der Differenz im Vergleich zu einem oder mehreren vorherigen Werten basiert, oder auf der Abweichung von Daten zwischen aufeinanderfolgenden Zeitperioden basiert,
- eine Anordnung, die so konfiguriert ist, dass sie die Daten der Zeitperiode(n) qualifiziert, wenn der Benutzer nachweislich wach und unbeweglich ist, und wenn der Artefakt-Prozentsatz unter einem vorbestimmten Wert liegt,
- eine Anordnung, die so konfiguriert ist, dass sie aus den qualifizierten Daten Herzfrequenzparameter berechnet, und
- eine Anordnung, die so konfiguriert ist, dass sie eine Funktion auf die Herzfrequenzparameter anwendet, um die Annäherung der minHF zu erhalten, wobei die Funktion das Erhalten eines Kandidaten für eine minHF und eines niedrigsten berechneten HR-Wertes aus den qualifizierten Zeitperioden von mehr als zwei aufeinanderfolgenden Zeitperioden und das Auswählen des Minimums von: dem Kandidaten für eine minHF und dem niedrigsten berechneten HF-Wert als die Annäherung der minHF umfasst.

10. Eine Vorrichtung nach Anspruch 9, umfassend eine oder mehrere Anordnung(en), die zur Durchführung des Verfahrens nach einem der Ansprüche 2-8 konfiguriert ist/sind.

11. Ein Computerprogrammprodukt zum Bereitstellen einer Annäherung an den minimalen Herzfrequenzwert, das ausführbare Anweisungen umfasst, die, wenn sie von einem Prozessor ausgeführt werden, so beschaffen sind, dass sie das Verfahren nach einem der Ansprüche 1-8 implementieren.

**Revendications**

1. Procédé mis en œuvre par ordinateur destiné à fournir une approximation d'une fréquence cardiaque minimale, (minHR) à partir de données de fréquence cardiaque collectées d'un utilisateur, le procédé comprenant

a) le calcul, à partir du signal de battement cardiaque collecté auprès d'un utilisateur, d'une valeur de fréquence cardiaque (HR) et d'un pourcentage d'artefact pour une ou plusieurs périodes de temps du signal de battement cardiaque collecté,
b) la qualification de données de la ou des périodes pour lesquelles,

o il est vérifié que l'utilisateur est éveillé et immobile, dans lequel l'utilisateur est vérifié comme étant éveillé sur la base d'au moins l'un parmi : des niveaux de variabilité de la fréquence cardiaque, l'écart de la variabilité de HR, le niveau de la fréquence cardiaque, la variabilité du niveau de la fréquence cardiaque, la valeur de fréquence cardiaque minimum actuelle, des données de mouvement, des entrées d'utilisateur et des données fournies par une autre source, et dans lequel il est vérifié que l'utilisateur est immobile sur la base d'au moins l'un parmi des données de mouvement, des niveaux de consommation d'oxygène (VO2), une consommation d'oxygène après l'exercice (EPOC), un pourcentage de la consommation maximale d'oxygène (%VO2max) et un état d'activité physique, et
o le pourcentage d'artefact est inférieur à une valeur prédéterminée, dans lequel le pourcentage d'artefact comprend une comparaison de valeurs d'intervalle entre battements, éventuellement dans lequel une valeur seuil est prédéterminée, ou basée sur une différence par rapport à une ou plusieurs valeurs précédentes, ou basée sur un écart de données entre des périodes successives,

et la disqualification de données des autres périodes de temps,

c) le calcul de paramètres de fréquence cardiaque à partir des données qualifiées,

d) l'application d'une fonction aux paramètres de fréquence cardiaque afin d'obtenir l'approximation de minHR, dans lequel la fonction comprend l'obtention d'un candidat pour une minHR et d'une valeur HR calculée la plus basse à partir des périodes de temps qualifiées de plus de deux périodes de temps successives, et la sélection du minimum parmi : le candidat pour une minHR et la valeur HR calculée la plus basse comme approximation de la minHR.

2. Procédé selon l'une quelconque des revendications précédentes, dans lequel les paramètres HR comprennent au moins l'un parmi la moyenne, l'écart type, la valeur minimale et la valeur maximale de HR.

3. Procédé selon l'une quelconque des revendications précédentes, dans lequel les données de fréquence cardiaque comprennent des données d'intervalle entre battements et le procédé comprend le calcul d'une variation de fréquence cardiaque (HRV) sur la base des données d'intervalle entre battements, et les paramètres HR comprennent au moins l'un parmi : une moyenne de HRV, un écart type de HRV, une valeur minimale de HRV et une valeur maximale de HRV.

4. Procédé selon l'une quelconque des revendications précédentes, dans lequel la fonction comprend des paramètres prédéterminés pour les limites supérieure et inférieure pour la valeur HR et la variabilité HR ; et éventuellement la limitation du candidat minHR entre les limites supérieure et inférieure prédéterminées.

5. Procédé selon l'une quelconque des revendications précédentes, comprenant la réception d'informations de base d'un utilisateur, dans lequel les informations de base comprennent des informations spécifiques à l'utilisateur, éventuellement l'âge et le sexe.

6. Procédé selon l'une quelconque des revendications précédentes, dans lequel le pourcentage d'artefact est déterminé à partir de la période de temps précédente et/ou à partir de valeurs moyennes des périodes de temps séquentielles.

7. Procédé selon l'une quelconque des revendications précédentes, dans lequel les données de fréquence cardiaque provenant d'un utilisateur comprennent la collecte de données à partir de points de périodes de temps séquentielles via une mesure en ligne.

8. Procédé selon l'une quelconque des revendications précédentes, dans lequel les données de fréquence cardiaque provenant d'un utilisateur comprennent des données enregistrées, précédemment collectées, éventuellement segmentées.

9. Appareil pour fournir une fréquence cardiaque minimale approximative (minHR) à partir de données de fréquence cardiaque collectées d'un utilisateur, l'appareil comprenant

- un agencement configuré pour calculer, à partir du signal de battement cardiaque collecté auprès d'un utilisateur, une valeur de fréquence cardiaque (HR) et un pourcentage d'artefact pour une ou plusieurs périodes de temps du signal de battement cardiaque collecté,

- un agencement configuré pour vérifier que l'utilisateur est éveillé et immobile pendant la période de temps, dans lequel il est vérifié que l'utilisateur est éveillé sur la base d'au moins l'un parmi : des niveaux de variabilité de la fréquence cardiaque, l'écart de la variabilité de HR, le niveau de la fréquence cardiaque, le niveau de variabilité de la fréquence cardiaque, la valeur de fréquence cardiaque minimale actuelle, les données de mouvement, les entrées de l'utilisateur et les données fournies par une autre source, et dans lequel il est vérifié que l'utilisateur est immobile sur la base d'au moins l'un parmi des données de mouvement, des niveaux de consommation d'oxygène (VO2), une consommation d'oxygène post-exercice (EPOC), un pourcentage de consommation maximale d'oxygène (%VO2max) et état un d'activité physique,

- un agencement configuré pour calculer un pourcentage d'artefact pour la période de temps, dans lequel le pourcentage d'artefact comprend une comparaison de valeurs d'intervalle entre battements, dans lequel éventuellement une valeur seuil est prédéterminée, ou basée sur une différence par rapport à une ou plusieurs valeurs précédentes, ou basée sur un écart de données entre périodes successives,

- un agencement configuré pour qualifier des données de la ou des périodes de temps, s'il est vérifié que l'utilisateur est éveillé et immobile ; et si le pourcentage d'artefact est inférieur à une valeur prédéterminée,

- un agencement configuré pour calculer des paramètres de fréquence cardiaque à partir des données qualifiées, et
- un agencement configuré pour appliquer une fonction aux paramètres de fréquence cardiaque, afin d'obtenir l'approximation de minHR, dans lequel la fonction comprend l'obtention d'un candidat pour une minHR et d'une valeur de HR calculée la plus basse à partir des périodes de temps qualifiées de plus de deux périodes de temps successives, et la sélection du minimum parmi : le candidat pour un minHR et la valeur HR calculée la plus basse comme approximation de minHR.

10. Appareil selon la revendication 9, comprenant un ou plusieurs agencements configurés pour mettre en œuvre le procédé selon l'une quelconque des revendications 2 à 8.

11. Produit de programme informatique destiné à fournir une approximation d'une valeur de fréquence cardiaque minimale, comprenant des instructions exécutables qui, lorsqu'elles sont exécutées par un processeur, sont programmées pour mettre en œuvre le procédé selon l'une quelconque des revendications 1 à 8.

| Receiving background information | 101 |

| Collecting heart rate data | 102 |

| Evaluating data | 103 |

| Estimating variables | 104 |

| Evaluating data of time period(s) | 105 |

Fig. 1

```
┌─────────────────────────────────────┐
│ (Receiving background information)   │──── 201
└─────────────────────────────────────┘
                  │
┌─────────────────────────────────────┐
│          Collecting data            │──── 202
└─────────────────────────────────────┘
                  │
┌─────────────────────────────────────┐
│            Evaluating               │──── 203
└─────────────────────────────────────┘
                  │
             ╱─────────╲
            ╱  User status: ╲──── 204
            ╲   awake?      ╱
             ╲─────────╱
                  │
             ╱─────────╲
            ╱  User status: ╲──── 205
            ╲   immobile?   ╱
             ╲─────────╱
                  │
             ╱─────────╲
            ╱ Data quality? ╲──── 206
            ╲              ╱
             ╲─────────╱
                  │
┌─────────────────────────────────────┐
│       Calculating parameters        │──── 207
└─────────────────────────────────────┘
                  │
┌─────────────────────────────────────┐
│         Applying a function         │──── 208
└─────────────────────────────────────┘
```

Fig. 2a

```
┌─────────────────────────────────────┐
│   Candidate for approximated minHR   │──── 2081
└─────────────────────────────────────┘
                  │
┌─────────────────────────────────────┐
│         Comparing to limits         │──── 2082
└─────────────────────────────────────┘
                  │
┌─────────────────────────────────────┐
│     Min (candidate, current value)  │──── 2083
└─────────────────────────────────────┘
```

Fig. 2b

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015189304 A
- US 20140088444 A1